# EUROPEAN PATENT SPECIFICATION

(11) **EP 2 480 212 B1**
(45) Date of publication and mention of the grant of the patent: **06.07.2016**
(21) Application number: 10757068.1
(22) Date of filing: 20.09.2010
(51) Int. Cl.: A61K 9/16, A61K 9/20, A61K 31/196, A61K 9/24, A61K 9/26

(54) **Oral pharmaceutical composition comprising diclofenac**
Orale pharmazeutische Zusammensetzung mit Diclofenac
Composition pharmaceutique orale contenant du diclofenac

(30) Priority: 25.09.2009 EP 09171309
(43) Date of publication of application: 01.08.2012
(73) Proprietor: Novartis Consumer Health S.A., 1197 Prangins (CH)
(72) Inventor: SHAW, Lance, CH-1260 Prangins (CH)
(74) Representative: Thompson, Clive Beresford
(86) International application number: PCT/EP2010/063782
(87) International publication number: WO 2011/036114

(56) References cited:
- EP-A1- 0 255 002
- EP-A1- 0 324 981
- EP-A1- 0 383 967
- EP-A1- 1 159 960
- WO-A1-03/074033
- DATABASE WPI Week 198629 Thomson Scientific, London, GB; AN 1986-184977 XP002570478 & JP 61 044811 A (SS PHARMACEUTICAL KK) 4 March 1986 (1986-03-04)

## Description

The invention concerns the oral administration of the non-steroidal anti-inflammatory drug diclofenac.

More particularly, it was an object of the present invention to provide an oral dosage form for the treatment of specific conditions which, on the one hand, have to be called sub-chronic because they typically may last for several days, but which, on the other hand, typically have a high initial pain level so that the patient expects very rapid pain relief, such as backache, morning stiffness; muscular pain, e.g. muscle twinges; chronic body pain, soft tissue injuries or sports injuries.

A further object of the invention was that a said sub-chronic condition could be effectively treated over several days by the patient taking said oral dosage form no more than twice a day only. Moreover, said oral dosage form should contain diclofenac in a relatively low dosage (50 mg or, preferably, less than 50 mg) so as to have the highest possible safety profile and allow self-medication by the patient, if desired.

Said requirements specification is quite different to that of conventional oral diclofenac modified release products which are typically aiming at truly chronic conditions, such as osteoarthritis or rheumatoid arthritis, where the patient is looking for satisfaction over a much longer timeframe.

In US patent 4 948 581, a long acting preparation comprising diclofenac sodium is disclosed, which essentially comprises a rapidly soluble component consisting essentially of diclofenac sodium and an enteric component consisting essentially of diclofenac sodium and an enteric coating therefore, especially methacrylic acid-methyl methacrylate copolymers- The weight ratio of rapidly soluble diclofenac sodium to diclofenac sodium in the enteric coating component recommended throughout said patent and exemplified in all of the examples is 3:7. Some speculation that said ratio may also be in the range of from 6:4 to 2:8 can be found. The problem to be solved by US patent 4 948 581 is described as providing "a long acting preparation which can sustain the action of diclofenac sodium in safe over an extended period of time", thus an improved sustained release preparation based on enteric coating of the active ingredient. In line with this, classical chronic indications like "rheumarthritis" (Experiment 5) or postoperative pain (Experiment 6) are addressed. From test results in Table 9 it can be seen that a significant difference in favor of the preparations of US patent 4 948 581 could only be observed after long term treatment (6h, 8h, 10h), when compared to a conventional diclofenac preparation.

In EP 1,159,960 A1, a two layered tablet with fast and slow release of diclofenac potassium salt is disclosed, see Example 5. However, said tablet was designed for the classical chronic indications of diclofenac, as can be seen from the total dose of 100 mg used. In clear contrast thereto, the tablets of the present invention have been designed for sub-chronic conditions (see further details below) and limited to a maximum amount of 50 mg of diclofenac potassium. With said lower total amounts of drug it was much more critical to incorporate a sufficient immediate-release portion into the tablet than with a 100 mg tablet. EP 1,159,960 A1 simply did not teach what was required for a 50 mg tablet to treat sub-chronic conditions.

In WO 03/074,033 A1, a sustained/controlled release formulation of diclofenac sodium with reduced risk of dose dumping is disclosed. Said formulation comprises two components, namely (a) diclofenac sodium combined with a water-insoluble but water-permeable polymer and (b) diclofenac sodium combined with a hydrophobic material.

In EP 383,967 A1, long acting diclofenac sodium preparations are disclosed. The concept followed to obtain prolonged action of the drug is to provide diclofenac sodium with an enteric coating. The downside of using an enteric coating, however, is that the drug will be released in the higher pH region of the gastro-intestinal tract. According to the pKa value of the drug this will result in less absorption as the drug is more ionized at higher pH values.

To achieve the goals of the present invention as outlined above, a lot of experimentation was necessary. It turned out that inter alia it was necessary to replace diclofenac sodium with diclofenac potassium, moreover the use of an enteric coating material as main component to delay the release of diclofenac had to be avoided.

Therefore, the present invention concerns a pharmaceutical composition for oral administration comprising diclofenac potassium in a total amount of from 25 to 50 mg, which composition comprises (a) an immediate-release portion of diclofenac potassium that comprises 33 to 67% of said total amount and (b) a sustained-release portion of diclofenac potassium that comprises 67 to 33% of said total amount.

Said pharmaceutical composition can be any orally administered dosage form, e.g. a monolithic one, e.g. a tablet or a capsule, or a multiparticulate one, e.g. granules, pellets or microspheres. Multiparticulate compositions may be further processed to form a monolithic dosage form. Preferably, said pharmaceutical composition is in the form of a tablet.

Preferably, said pharmaceutical composition comprises diclofenac potassium in a total amount of from 25 to 40 mg, especially of from 30 to 40 mg.

All percentages given are weight-%, unless indicated otherwise.

Preferred are said pharmaceutical compositions, which comprise (a) an immediate-release portion of diclofenac potassium that comprises 40 to 60% - especially 45 to 60%, more especially 50-60% and in particular 55% - of the total amount of diclofenac potassium and (b) a sustained-release portion of diclofenac potassium that comprises 60 to 40% - especially 55 to 40%, more especially 50-40% and in particular 45% - of the total amount of diclofenac potassium.

The sustained release properties can be imparted to said sustained-release portion (b), for example, through the utilization of technologies with pH dependent or pH independent mechanisms, preferably through the utilization of pH dependent or pH independent mechanisms excluding enteric coating of diclofenac potassium, and in particular through the utilization of pH independent mechanisms, such as hydrophilic or hydrophobic matrices.

Thus, it is preferred that said sustained-release portion of diclofenac potassium (b) is devoid of an enteric coating of diclofenac potassium.

In another embodiment of the invention, said sustained-release portion of diclofenac potassium (b) is devoid of enteric coating materials or does comprise enteric coating materials only in amounts essentially not causing any delayed release.

In a further embodiment of the invention, the pharmaceutical compositions for oral administration according to the present invention are devoid of any methacrylic acid copolymers, such as Eudragit® RS, Eudragit® RL, Eudragit® NE, Eudragit® NM, Eudragit® S or Eudragit® L materials (all available from Rohm Pharma Polymers).

Preferably, said sustained-release portion (b) comprises hydroxypropyl methylcellulose.

More preferably, the percentage (w/w) of hydroxypropyl methylcellulose in said sustained-release portion (b) of the pharmaceutical composition is from 35 up to 60%, and in particular from 35 up to 50%.

Expressing the preferred amounts of hydroxypropyl methylcellulose in a different way, the weight ratio of hydroxypropyl methylcellulose to diclofenac potassium in said sustained-release portion (b) preferably is from 0.7 to 6, and in particular from 2 to 5.

Optionally, said immediate-release portion (a) further comprises a superdisintegrant, e.g. sodium starch glycolate, croscarmellose sodium or crospovidone, in particular sodium starch glycolate.

The pharmaceutical compositions of the invention can be manufactured in a manner known per se. For example, said portions (a) and (b) can be manufactured separately from each other and then brought together so that a pharmaceutical composition of the invention is obtained. In a specific embodiment, said portions (a) and (b) can be pressed together, e.g. to form a multi-layer tablet.

In Table 1 (see below), the in-vitro dissolution profiles of some tablets of the invention are disclosed.

Preferred are those pharmaceutical compositions of the invention, wherein - when tested according to the following in vitro dissolution method and conditions: USP XXXII /EP Paddle apparatus, 50 rpm, buffer 0.01 M KH2PO4 - buffer pH 6.8, temperature 37°C - 36 to 68% of the total active substance has dissolved after 15 minutes (t = 15 min) and 51 to 79% of the total active substance after two hours (t = 2h).

More preferred are those, wherein 44 to 60% - especially 50 to 60% - of the total active substance has dissolved after 15 minutes (t = 15 min), and 51 to 79% - especially 55 to 75% - of the total active substance after two hours (t = 2h).

Preferred sub-chronic conditions with the potential of having a high initial pain level are: (1) backache; or (2) morning stiffness; or (3) muscular pain, e.g. muscle twinges.

The beneficial properties of the pharmaceutical compositions of the invention are demonstrated e.g. by the following tests:
Plasma levels of diclofenac: The plasma levels of diclofenac are measured in humans (samples of venous blood) at different time intervals (e.g. 4h and 6h) after oral administration (e.g. twice daily) of a composition of the invention. Even 12h after oral administration, diclofenac plasma levels are detectable proving the slow/sustained release properties of the composition.

Food Effect Study: In a single dose 3-way randomized cross-over design of "composition of the invention fasted" versus "fed" versus "immediate release diclofenac K fasted" in 36 subjects, it is demonstrated that food intake has no effect on the pharmacokinetic (PK) profile of the composition of the invention, and therefore does not impact on its efficacy either. Venous blood samples are collected before dosing and then at different time intervals after drug administration (e.g. 1 h, 2h and 4h).

Dental Pain Study [single dose (12h) and two dose (24h)]: In a 24h randomized, double blind, placebo controlled study with two treatment groups of 100 subjects each (composition of the invention versus placebo) fast onset and long lasting efficacy of the composition of the invention is demonstrated in third molar extraction.

Back Pain Study (multiple dose): In a 7-day, multiple doses, randomized, double blind, parallel group, placebo controlled study with three treatment groups of 100 subjects each (composition of the invention versus reference product, immediate release diclofenac K, versus placebo) efficacy of the composition of the invention (long lasting) and higher convenience of twice a day dosing only (as compared to reference regimen) are demonstrated.

The following examples are intended to illustrate the invention. "IR" means "immediate release", and "SR" sustained release. All amounts indicated are given in mg.

**Examples 1-3: Tablets comprising 37.5 mg Diclofenac potassium with immediate-release and sustained-release dual action**

| | Comparative Example 1 | Example 2 | Example 3 |
|---|---|---|---|
| IR portion of the tablet | | | |
| Diclofenac potassium USP | 6.25 | 12.5 | 15 |
| Weight ratio IR Diclofenac K/ Total Diclofenac K | 16.7% | 33.3% | 40% |
| Lactose monohydrate | 4 | 8 | 9.6 |
| Sodium starch glycolate | 3 | 6 | 7.2 |
| Microcrystalline cellulose | 8.125 | 16.25 | 19.52 |
| Lubricating materials | | | |
| Colloidal anhydrous silica | 0.5 | 1 | 1.2 |
| Magnesium stearate | 0.5 | 1 | 1.2 |
| Maize starch (Veg grade) | 2.5 | 5 | 6 |
| Red ferric oxide (E172) | 0.125 | 0.25 | 0.28 |
| Weight of the IR portion | 25 | 50 | 60 |

| SR portion of the tablet | | | |
|---|---|---|---|
| Diclofenac potassium USP | 31.25 | 25 | 22.5 |
| Weight ratio SR Diclofenac K/ Total Diclofenac K | 83.3% | 66.7% | 60% |
| Lactose monohydrate | 12.25 | 9.5 | 9 |
| Hydroxypropyl methylcellulose | 36 | 29 | 26 |

| Extragranular materials | | | |
|---|---|---|---|
| Magnesium stearate | 0.5 | 0.5 | 0.5 |
| Purified water | q.s. | q.s. | q.s. |
| Weight of the SR portion | 80 | 64 | 58 |
| Total tablet weight | 105 | 114 | 118 |

**Examples 4-6: Tablets comprising 37.5 mg Diclofenac potassium with immediate-release and sustained-release dual action**

| | Example 4 | Example 5 | Example 6 |
|---|---|---|---|
| IR portion of the tablet | | | |
| Diclofenac potassium USP | 12.5 | 16.88 | 12.50 |
| Weight ratio IR Diclofenac K/ Total Diclofenac K | 33.3% | 45% | 33.3% |
| Lactose monohydrate | 8 | 3.62 | 8.00 |
| Sodium starch glycolate | 6 | 6.00 | 6.00 |
| Microcrystalline cellulose | 16.26 | 16.26 | 16.26 |

| Lubricating materials | | | |
|---|---|---|---|
| Colloidal anhydrous silica | 1 | 1.00 | 1.00 |
| Magnesium stearate | 1 | 1.00 | 1.00 |
| Maize starch (Veg grade) | 5 | 5.00 | 5.00 |
| Red ferric oxide (E172) | 0.24 | 0.24 | 0.24 |
| Weight of the IR portion | 50 | 50.00 | 50.00 |

| SR portion of the tablet | | | |
|---|---|---|---|
| Diclofenac potassium USP | 25 | 20.62 | 25.00 |
| Weight ratio SR Diclofenac K/ Total Diclofenac K | 66.7% | 55% | 66.7% |
| Lactose monohydrate | 53 | 70.38 | 94.46 |
| Hydroxypropyl methylcellulose | 49 | 73.00 | 71.54 |

| Extragranular materials | | | |
|---|---|---|---|
| Magnesium stearate | 1 | 1.00 | 1.0 |
| Purified water | q.s. | q.s. | q.s. |
| Weight of the SR portion | 128 | 165.00 | 192.00 |
| Total tablet weight | 178 | 215.00 | 242.00 |

**Examples 7-9: Tablets comprising 37.5 mg Diclofenac potassium with immediate-release and sustained-release dual action**

| | Example 7 | Example 8 | Example 9 |
|---|---|---|---|
| IR portion of the tablet | | | |
| Diclofenac potassium USP | 12.50 | 20.625 | 22.50 |
| Weight ratio IR Diclofenac K/ Total Diclofenac K | 33.3% | 55% | 60% |
| Lactose monohydrate | 8.00 | 4.42 | 4.83 |
| Sodium starch glycolate | 6.00 | 7.33 | 8.00 |
| Microcrystalline cellulose | 16.26 | 19.87 | 21.67 |

| Lubricating materials | | | |
|---|---|---|---|
| Colloidal anhydrous silica | 1.00 | 1.00 | 1.00 |
| Magnesium stearate | 1.00 | 1.00 | 1.00 |
| Maize starch (Veg grade) | 5.00 | 5.00 | 5.00 |
| Red ferric oxide (E172) | 0.24 | 0.24 | 0.24 |
| Weight of the IR portion | 50.00 | 59.485 | 64.24 |

| SR portion of the tablet | | | |
|---|---|---|---|
| Diclofenac potassium USP | 25.00 | 16.875 | 15.00 |
| Weight ratio SR Diclofenac K/ Total Diclofenac K | 66.7% | 45% | 40% |
| Lactose monohydrate | 95.92 | 57.58 | 51.19 |
| Hydroxypropyl methylcellulose | 70.08 | 59.73 | 53.09 |

| Extragranular materials | | | |
|---|---|---|---|
| Magnesium stearate | 1.00 | 1.00 | 1.0 |
| Purified water | q.s. | q.s. | q.s. |
| Weight of the SR portion | 192.00 | 135.185 | 120.28 |
| Total tablet weight | 242.00 | 194.67 | 184.52 |

**Examples 10-12: Tablets comprising 25 mg Diclofenac potassium with immediate-release and sustained-release dual action**

| | Example 10 | Example 11 | Example 12 |
|---|---|---|---|
| IR portion of the tablet | | | |
| Diclofenac potassium USP | 8.25 | 13.75 | 15.00 |
| Weight ratio IR Diclofenac K/ Total Diclofenac K | 33.3% | 55% | 60% |
| Lactose monohydrate | 8.00 | 4.42 | 4.83 |
| Sodium starch glycolate | 6.00 | 7.33 | 8.00 |
| Microcrystalline cellulose | 16.26 | 19.87 | 21.67 |

| Lubricating materials | | | |
|---|---|---|---|
| Colloidal anhydrous silica | 1.00 | 1.00 | 1.00 |
| Magnesium stearate | 1.00 | 1.00 | 1.00 |
| Maize starch (Veg grade) | 5.00 | 5.00 | 5.00 |
| Red ferric oxide (E172) | 0.24 | 0.24 | 0.24 |
| Weight of the IR portion | 45.75 | 52.61 | 56.74 |

| SR portion of the tablet | | | |
|---|---|---|---|
| Diclofenac potassium USP | 16.75 | 11.25 | 10.00 |
| Weight ratio SR Diclofenac K/ Total Diclofenac K | 66.7% | 45% | 40% |
| Lactose monohydrate | 95.92 | 57.58 | 51.19 |
| Hydroxypropyl methylcellulose | 70.08 | 56.00 | 50.00 |

| Extragranular materials | | | |
|---|---|---|---|
| Magnesium stearate | 1.00 | 1.00 | 1.0 |
| Purified water | q.s. | q.s. | q.s. |
| Weight of the SR portion | 183.75 | 125.83 | 112.19 |
| Total tablet weight | 229.5 | 178.44 | 168.93 |

**Examples 13-15: Tablets comprising 50 mg Diclofenac potassium with immediate-release and sustained-release dual action**

| | Example 13 | Example 14 | Example 15 |
|---|---|---|---|
| IR portion of the tablet | | | |
| Diclofenac potassium USP | 16-65 | 27.50 | 30.00 |
| Weight ratio IR Diclofenac K/ Total Diclofenac K | 33.3% | 55% | 60% |
| Lactose monohydrate | 8.00 | 4.42 | 4.83 |
| Sodium starch glycolate | 6.00 | 7.33 | 8.00 |
| Microcrystalline cellulose | 16.26 | 39.87 | 41.67 |

| Lubricating materials | | | |
|---|---|---|---|
| Colloidal anhydrous silica | 1.00 | 1.00 | 1.00 |
| Magnesium stearate | 1.00 | 1.00 | 1.00 |
| Maize starch (Veg grade) | 5.00 | 5.00 | 5.00 |
| Red ferric oxide (E172) | 0.24 | 0.24 | 0.24 |
| Weight of the IR portion | 54.15 | 86.36 | 91.74 |

| SR portion of the tablet | | | |
|---|---|---|---|
| Diclofenac potassium USP | 33.35 | 22.50 | 20.00 |
| Weight ratio SR Diclofenac K/ Total Diclofenac K | 66.7% | 45% | 40% |
| Lactose monohydrate | 95.92 | 57.58 | 51.19 |
| Hydroxypropyl) methylcellulose | 70.08 | 59.73 | 53.09 |

| Extragranular materials | | | |
|---|---|---|---|
| Magnesium stearate | 1.00 | 1.00 | 1.0 |
| Purified water | q.s. | q.s. | q.s. |
| Weight of the SR portion | 200.35 | 140.81 | 125.28 |
| Total tablet weight | 254.5 | 227.17 | 217.02 |

The tablets of the Examples 1-15 are manufactured as follows.

Preparation of IR Portion: Pass each of diclofenac K, microcrystalline cellulose, lactose Monohydrate and sodium starch glycolate through a 40 mesh sieve. Mix uniformely diclofenac K, microcrystalline cellulose and lactose Monohydrate for 2 min. Add sodium starch glycolate and mix uniformly. Pass colloidal anhydrous silica through 40 mesh sieve, add to blend and mix uniformly. Pass the color Red ferric oxide along with starch through 100 mesh sieve. Mix with blend. Finally lubricate blend with magnesium stearate (40 mesh sieve).

Preparation of SR Portion: Pass each of diclofenac K, lactose monohydrate and hydroxypropyl methylcellulose through 40 mesh sieve. Mix diclofenac, lactose monohydrate and hydroxypropyl methylcellulose uniformly. Granulate the resulting blend with water. Pass the wet mass through 8 mesh screen. Dry the passed mass in a dryer (e.g. from company Retsch, Germany). Check the moisture content of the dried granules. Pass the dried granules through 16 mesh sieve. Check the density of dry granules. Lubricate the resulting blend with magnesium stearate (40 mesh sieve).

Preparation of bi-layer tablet: The IR and SR portions are compressed using a standard pharmaceutical process to form a bi-layer tablet using a bi-layer tablet press (e.g. from company Korsch, Germany), with one layer containing the IR portion and the other layer containing the SR portion.

The tablets of the invention described in the examples all have similar bi-phasic in-vitro dissolution profiles. The initial phase shows a rapid dissolution rate which can be measured at for example the 15 minute timepoint. The second phase consists of a slower sustained dissolution rate which can be observed after 2 hours where incomplete dissolution is observed. The actual percentage dissolved during the rapid and slower sustained phases are dependent on the ratio of diclofenac potassium in the (a) immediate release portion and (b) sustained release portion.

**Table 1: in-vitro Dissolution profiles of some tablets of the invention**

| time [min] | Amount of diclofenac K dissolved (in % of total diclofenac K amount, 37.5 mg) | | | | | |
|---|---|---|---|---|---|---|
| | Tablet of Comparative Example 1 | Tablet of Example 2 | Tablet of Example 3 | Tablet of Example 4 | Tablet of Example 5 | Tablet of Example 6 |
| 0 | 0 | 0 | 0 | 0 | 0 | 0 |
| 15 | 25 | 41 | 45 | 37 | 41 | 41 |
| 30 | 31 | 47 | 51 | 42 | 49 | 47 |
| 60 | n.m. | 57 | 59 | 47 | 57 | 50 |
| 120 | n.m. | 74 | 75 | 57 | 67 | 54 |
| 240 | 85 | 95 | 93 | 77 | 81 | 68 |
| 360 | 96 | 100 | 98 | 85 | 90 | 78 |
| 600 | 105 | 104 | 102 | 98 | n.m. | n.m. |

| | | | | | | |
|---|---|---|---|---|---|---|
| n.m. = not measured | | | | | | |

## Claims

1. A pharmaceutical composition for oral administration comprising diclofenac potassium in a total amount of from 25 to 50 mg, which composition comprises (a) an immediate-release portion of diclofenac potassium that comprises 33 to 67% of said total amount and (b) a sustained-release portion of diclofenac potassium that comprises 67 to 33% of said total amount, wherein the sustained-release portion (b) comprises hydroxypropyl methylcellulose and wherein the percentage (w/w) of hydroxypropyl methylcellulose in said sustained-release portion (b) is from 35 up to 60%.

2. A composition according to claim 1 wherein the percentage (w/w) of hydroxypropyl methylcellulose in the sustained-release portion (b) is from 35 up to 50%.

3. A composition according to claim 1 or 2 comprising diclofenac potassium in an amount of from 25 to 40 mg.

4. A composition according to any one of claims 1-3, which is in the form of a tablet.

5. A composition according to any one of claims 1-4, which comprises (a) an immediate-release portion of diclofenac potassium that comprises 40 to 60% of the total amount of diclofenac potassium and (b) a sustained-release portion of diclofenac potassium that comprises 60 to 40% of the total amount of diclofenac potassium.

6. A composition according to any one of claims 1-4, which comprises (a) an immediate-release portion of diclofenac potassium that comprises 55 to 60% of the total amount of diclofenac potassium and (b) a sustained-release portion of diclofenac potassium that comprises 45 to 40% of the total amount of diclofenac potassium.

7. A composition according to claim 1, wherein the weight ratio of hydroxypropyl methylcellulose to diclofenac potassium in said sustained-release portion (b) is of from 0.7 up to 6.

8. A composition according to any one of claims 1-7, wherein the immediate-release portion (a) comprises a superdisintegrant.

9. A composition according to any one of claims 1-8, which is in the form of a 25-40 mg tablet, for use in b.i.d. (twice daily) treatment of sub-chronic conditions with the potential of having a high initial pain level selected from the group consisting of backache, morning stiffness, muscular pain such as muscle twinges, chronic body pain, soft tissue injuries and sports injuries.

10. A composition according to any one of claims 1-8, which is in the form of a 30-40 mg, especially 37.5 mg, tablet, for use in b.i.d. (twice daily) treatment of sub-chronic conditions with the potential of having a high initial pain level selected from the group consisting of backache, morning stiffness, muscular pain such as muscle twinges, chronic body pain, soft tissue injuries and sports injuries.

11. A composition according to any one of claims 1-10, wherein said sustained-release portion of diclofenac potassium (b) is devoid of enteric coating materials or does comprise enteric coating materials only in amounts essentially not causing any delayed release.

12. A composition according to any one of claims 1-10, wherein said sustained-release portion of diclofenac potassium (b) is devoid of an enteric coating of diclofenac potassium.

13. A composition according to any one of claims 1-12, which is devoid of any methacrylic acid copolymers.

## Patentansprüche

1. Pharmazeutische Zusammensetzung zur oralen Verabreichung, umfassend Diclofenac-Kalium in einer Gesamtmenge von 25 bis 50 mg, wobei die Zusammensetzung umfasst: (a) einen Anteil an Diclofenac-Kalium mit sofortiger Freisetzung, welcher 33 bis 67% der Gesamtmenge ausmacht, und (b) einen Anteil an Diclofenac-Kalium mit verzögerter Freisetzung, welcher 67 bis 33% der Gesamtmenge ausmacht, wobei der Anteil mit verzögerter Freisetzung (b) Hydroxypropylmethylzellulose umfasst, und wobei der Prozentanteil (w/w) an Hydroxypropylmethylzellulose in dem Anteil mit verzögerter Freisetzung (b) von 35 bis zu 60% beträgt.

2. Zusammensetzung gemäß Anspruch 1, wobei der Prozentanteil (w/w) an Hydroxypropylmethylzellulose in dem Anteil mit verzögerter Freisetzung (b) von 35 bis zu 50% beträgt.

3. Zusammensetzung gemäß Anspruch 1 oder 2, welche Diclofenac-Kalium in einer Menge von 25 bis 40 mg umfasst.

4. Zusammensetzung gemäß irgendeinem der Ansprüche 1-3, welche in Form einer Tablette vorliegt.

5. Zusammensetzung gemäß irgendeinem der Ansprüche 1-4, welche umfasst: (a) einen Anteil an Diclofenac-Kalium mit sofortiger Freisetzung, welcher 40 bis 60% der Gesamtmenge an Diclofenac-Kalium ausmacht, und (b) einen Anteil an Diclofenac-Kalium mit verzögerter Freisetzung, welcher 60 bis 40% der Gesamtmenge an Diclofenac-Kalium ausmacht.

6. Zusammensetzung gemäß irgendeinem der Ansprüche 1-4, welche umfasst: (a) einen Anteil an Diclofenac-Kalium mit sofortiger Freisetzung, welcher 55 bis 60% der Gesamtmenge an Diclofenac-Kalium ausmacht, und (b) einen Anteil an Diclofenac-Kalium mit verzögerter Freisetzung, welcher 45 bis 40% der Gesamtmenge an Diclofenac-Kalium ausmacht.

7. Zusammensetzung gemäß Anspruch 1, wobei das Gewichtsverhältnis von Hydroxypropylmethylzellulose zu Diclofenac-Kalium in dem Anteil mit verzögerter Freisetzung (b) von 0,7 bis zu 6 beträgt.

8. Zusammensetzung gemäß irgendeinem der Ansprüche 1-7, wobei der Anteil mit sofortiger Freisetzung (a) ein Super-Sprengmittel umfasst.

9. Zusammensetzung gemäß irgendeinem der Ansprüche 1-8, welche in Form einer 25-40 mg-Tablette vorliegt, zur Verwendung in b.i.d.- (zweimal täglicher) Behandlung subchronischer Zustände, die potentiell ein hohes initiales Schmerzniveau aufweisen, ausgewählt aus der Gruppe, bestehend aus Rückenschmerzen, Morgensteifigkeit, Muskelschmerzen, wie Muskelstechen, chronische Körperschmerzen, Weichgewebeverletzungen und Sportverletzungen.

10. Zusammensetzung gemäß irgendeinem der Ansprüche 1-8, welche in Form einer 30-40 mg-, insbesondere einer 37,5 mg-Tablette vorliegt, zur Verwendung in b.i.d.-(zweimal täglicher) Behandlung subchronischer Zustände, die potentiell ein hohes initiales Schmerzniveau aufweisen, ausgewählt aus der Gruppe, bestehend aus Rückenschmerzen, Morgensteifigkeit, Muskelschmerzen, wie Muskelstechen, chronischen Körperschmerzen, Weichgewebeverletzungen und Sportverletzungen.

11. Zusammensetzung gemäß irgendeinem der Ansprüche 1-10, wobei der Anteil an Diclofenac-Kalium mit verzögerter Freisetzung (b) keine enterischen Beschichtungsmaterialien aufweist oder enterische Beschichtungsmaterialien nur in Mengen aufweist, die im Wesentlichen keinerlei verzögerte Freisetzung verursachen.

12. Zusammensetzung gemäß irgendeinem der Ansprüche 1-10, wobei der Anteil an Diclofenac-Kalium mit verzögerter Freisetzung (b) keine enterische Beschichtung des Diclofenac-Kaliums aufweist.

13. Zusammensetzung gemäß irgendeinem der Ansprüche 1-12, welche keinerlei Methacrylsäure-Copolymere aufweist.

## Revendications

1. Composition pharmaceutique pour administration orale, comprenant du diclofénac de potassium en une quantité totale de 25 mg à 50 mg, ladite composition comprenant (a) une partie à libération immédiate de diclofénac de potassium qui comprend 33 % à 67 % de ladite quantité totale et (b) une partie à libération prolongée de diclofénac de potassium qui comprend 67 % à 33 % de ladite quantité totale, dans laquelle la partie à libération prolongée (b) comprend de l'hydroxypropylméthylcellulose et dans laquelle le pourcentage (en poids/poids) d'hydroxypropylméthyl-cellulose dans ladite partie à libération prolongée (b) est de 35 % à 60 %.

2. Composition selon la revendication 1, dans laquelle le pourcentage (en poids/poids) d'hydroxy-propylméthylcellulose dans ladite partie à libération prolongée (b) est de 35 % à 50 %.

3. Composition selon la revendication 1 ou 2, comprenant du diclofénac de potassium en une quantité de 25 mg à 40 mg.

4. Composition selon l'une quelconque des revendications 1 à 3, qui est sous la forme d'un comprimé.

5. Composition selon l'une quelconque des revendications 1 à 4, qui comprend (a) une partie à libération immédiate de diclofénac de potassium qui comprend 40 % à 60 % de la quantité totale de diclofénac de potassium et (b) une partie à libération prolongée de diclofénac de potassium qui comprend 60 % à 40 % de la quantité totale de diclofénac de potassium.

6. Composition selon l'une quelconque des revendications 1 à 4, qui comprend (a) une partie à libération immédiate de diclofénac de potassium qui comprend 55 % à 60 % de la quantité totale de diclofénac de potassium et (b) une partie à libération prolongée de diclofénac de potassium qui comprend 45 % à 40 % de la quantité totale de diclofénac de potassium.

7. Composition selon la revendication 1, dans laquelle le rapport pondéral entre l'hydroxypropyl-méthylcellulose et le diclofénac de potassium dans ladite partie à libération prolongée (b) est de 0,7 à 6.

8. Composition selon l'une quelconque des revendications 1 à 7, dans laquelle la partie à libération immédiate (a) comprend un superdélitant.

9. Composition selon l'une quelconque des revendications 1 à 8, qui est sous la forme d'un comprimé de 25 mg à 40 mg, pour son utilisation dans le traitement biquotidien (deux fois par jour) d'affections sous-chroniques ayant le potentiel de présenter un niveau initial élevé de douleur, choisies dans le groupe constitué par le mal de dos, la raideur matinale, une douleur musculaire telle que les élancements musculaires, une douleur corporelle chronique, les lésions des tissus mous et les lésions dues au sport.

10. Composition selon l'une quelconque des revendications 1 à 8, qui est sous la forme d'un comprimé de 30 mg à 40 mg, en particulier un comprimé de 37,5 mg, pour son utilisation dans le traitement biquotidien (deux fois par jour) d'affections sous-chroniques ayant le potentiel de présenter un niveau initial élevé de douleur, choisies dans le groupe constitué par le mal de dos, la raideur matinale, une douleur musculaire telle que les élancements musculaires, une douleur corporelle chronique, les lésions des tissus mous et les lésions dues au sport.

11. Composition selon l'une quelconque des revendications 1 à 10, dans laquelle ladite partie à libération prolongée de diclofénac de potassium (b) est dépourvue de matières d'enrobage entérique ou comprend des matières d'enrobage entérique seulement en des quantités ne provoquant essentiellement pas une libération retardée.

12. Composition selon l'une quelconque des revendications 1 à 10, dans laquelle ladite partie à libération prolongée de diclofénac de potassium (b) est dépourvue d'un enrobage entérique du diclofénac de potassium.

13. Composition selon l'une quelconque des revendications 1 à 12, qui est dépourvue de copolymères d'acide méthacrylique.
